# EUROPÄISCHE PATENTSCHRIFT

(11) **EP 0 584 715 B1**
(45) Veröffentlichungstag und Bekanntmachung des Hinweises auf die Patenterteilung: **23.12.1998**
(21) Anmeldenummer: 93113227.8
(22) Anmeldetag: 18.08.1993
(51) Int. Cl.: C12N 5/08, C12N 5/20, C12P 21/08, G01N 33/50, G01N 33/483

(54) **Verfahren zur Isolierung und Kultivierung von transformierten Zellen sowie Verwendung dieser Zellen zur Herstellung individuumsspezifischer Antikörper**
Process for isolation and culturing of transformed cells and use thereof for the preparation of antibodies against them
Pocédé pour l'isolation et la cultivation de cellules transformées et leur utilisation pour la préparation des anticorps contre ces cellules.

(30) Priorität: 26.08.1992 DE 4228389
(43) Veröffentlichungstag der Anmeldung: 02.03.1994
(73) Patentinhaber: Dr. Kübler GmbH, 81675 München (DE)
(72) Erfinder: Kübler, Ulrich, Dr., D-80538 München (DE); Hoffmann, Rainer, D-82256 Fürstenfeldbruck (DE)
(74) Vertreter: Nätebusch, Roderich

(56) Entgegenhaltungen:
- WO-A-89/05657
- WO-A-89/07445
- US-A- 5 112 298
- PROGRESS IN CLINICAL PATHOLOGY, Bd.3, 1970, NEW YORK, USA, Seiten 362-382; WEST ET AL:'CHAPTER 11,TUMOR EMBOLIZATION IN MAN.STUDIES OF CANCER CELLS IN THE BLOOD'
- DEVELOPMENTS IN ONCOLOGY, Bd.49, 1986, DEN HAAG, Seiten 251-271; GULLINO:'CONSIDERATIONS OF CLINICAL RELEVANCE ON THE METASTATIC PROCESS:A WORKING HYPOTHESIS'
- FILE SERVER STN KARLSRUHE,FILE MEDLINE ZUSAMMENFASSUNG 92162858 & J VET MED SCI, (1991 DEC) 53(6), 969-74
- FILE SERVER STN KARLSRUHE,FILE MEDLINE ZUSAMMENFASSUNG 86087294 & J IMMUNOL METHODS, (1985 DEC 27), 85(2), 353-61
- FILE SERVER STN KARLSRUHE,FILE MEDLINE ZUSAMMENFASSUNG 92061867 & AVIAN DIS, (1991 JUL-SEP), 35(3), 563-71
- FILE SERVER STN KARLSRUHE,FILE MEDLINE ZUSAMMENFASSUNG 84088101 & J CELL BIOCHEM, (1983), 21(4), 277-87
- FILE SERVER STN KARLSRUHE,FILE MEDLINE ZUSAMMENFASSUNG 86034611 & J CLIN INVEST, (1985 OCT), 76(4), 1649-56

## Beschreibung

Die Erfindung betrifft ein Verfahren zur Gewinnung transformierter Zellen aus dem Blutstrom eines menschlichen oder tierischen Individuums, ein Verfahren zur Kultivierung der so gewonnenen Zellen und die Herstellung von gegen diese Zellen gerichteten monoklonalen Antikörpern.

Über die Bedeutung von im Blutstrom zirkulierenden transformierten Zellen für die Ausbildung von Metastasen berichtet A.J. Salsbury in "Significance of Circulating Cancer Cells", William Heinemann Medical Books, New Aspects of Breast Cancer, Bd. 3, S. 245ff, 1977, daß diese im Blutstrom zirkulierenden Krebszellen in einem Zeitraum bis zu 5 - 6 Stunden nach ihrer Freisetzung - z.B. durch bestimmte operative und therapeutische Maßnahmen - noch keine Metastasen ausbilden können. Das Vorhandensein großer Mengen an Krebszellen allein genügt noch nicht für das Auftreten von Metastasen, die Krebszellen müßten zuerst den vaskulären Bereich verlassen. Dennoch wird die Verwendung von Mitteln vorgeschlagen, die einerseits die Ausbildung von Thromben als Ausgangspunkte für die Metastasenbildung aus dem Blutstrom verhindern und andererseits die malignen Zellen abtöten sollen.

Neuere Erkenntnisse (P.M. Gullino, Dev. Oncol., 49, 251-271 (1986)) legen jedoch nahe, daß zirkulierende Tumorzellen in den meisten Fällen, bereits lange bevor der Primärtumor klinisch nachgewiesen werden kann, im Blutstrom auftreten. Auch wenn nicht alle dieser transformierten Zellen zur Auslösung von Metastasen in der Lage sind, ist es erforderlich, die Ausbreitung von zirkulierenden Tumorzellen und damit das Metastasenwachstum so früh wie möglich zu bekämpfen. Der Grund, warum bislang noch keine eindeutige Korrelation zwischen der Anzahl transformierter Zellen im peripheren Blut und der Auslösung der Metastasenbildung aufgestellt werden konnte, liegt zu einem großen Teil in der Schwierigkeit des Nachweises der transformierten Zellen im Blutstrom.

Ferner ist bekannt, daß schon bei üblichen Diagnoseverfahren, wie Abtasten des soliden Tumors, und insbesondere bei bioptischen und operativen Eingriffen die Freisetzung transformierter Tumorzellen deutlich erhöht wird, was zu einer verstärkten Metastasenbildung führen kann.

Das Metastasierungs-Verhalten von soliden Tumoren in das Lymph- und/oder Kreislauf-System hinein ist bisher in vivo nur in eröffneten Blutgefäßen bei Operationen studiert worden. Zirkulierende Tumorzellen in diagnostisch oder gar therapeutisch verwertbarem Umfang wurden dabei nicht gewonnen. Das Metastasierungs-Verhalten von soliden Tumoren in den Kreislauf ließ sich daher nur in Tierversuchen oder beim Menschen nur postmortal durch den Pathologen untersuchen.

Man kennt eine Reihe von Verfahren, transformierte Zellen aus dem Blut zu gewinnen: chemische oder enzymatische Lyse von nicht-transformierten Zellen, beschleunigte Sedimentation roter Blutzellen, Flotations-, Zentrifugations- und magnetische Trennverfahren usw. (siehe Übersicht bei J.A.Fleming und J.W.Stewart, Journal of Clinical Pathology, 20, 145-151 (1967), und J.T.West und R.Hume, Progress in Clinical Pathology, Bd.3, Kap.11, 362-382 (1970)). Die bekannten Methoden führen meist nur zu geringen Ausbeuten und zeigen auch insbesondere den Mangel, daß man nicht feststellen konnte, ob eine transformierte Zelle aus dem peripheren Kreislauf zum Zeitpunkt ihrer Gewinnung vital war oder nicht. Trotzdem wird bis heute auf diese Methoden immer noch zurückgegriffen.

Aus EP-A- 0 443 599 ist bereits die Vewendung von karzizogenen Zellinien zur Herstellung monoklonaler Antikörper bekannt.

Transformierte Zellen lassen sich demnach mit einer Dichtegradienten-Zentrifugation, wie sie z.B. üblicherweise zur Isolierung von Lymphozyten in einem Ficoll-, Silikon-oder Albumingradienten eingesetzt wird, gewinnen. Hierfür sind jedoch große Blutmengen erforderlich, was für den Patienten sehr belastend ist. Ferner ist auch trotz eines hohen apparativen Aufwands die Ausbeute aufgrund der geringen Anzahl transformierter Zellen, die zudem durch das konventionelle Zentrifugationsverfahren leicht beschädigt und verunreinigt werden, äußerst niedrig. Daher ist ein Dichtegradienten-Zentrifugationsverfahren routinemäßig zur Gewinnung größerer Mengen von transformierten Zellen nicht einsetzbar. Eine Rückführung der übrigen Blutbestandteile, wie Plasma und rote Blutkörperchen, an den Patienten ist aufgrund der Verunreinigungen durch Gradientenmaterial praktisch nicht durchführbar. Für eine klinische Anwendung am Patienten sind die Zentrifugationsverfahren nicht geeignet.

Es existiert somit bislang kein einfach durchzuführendes und reproduzierbares Verfahren, das es erlaubt, auf nicht chirurgische Weise eventuell im peripheren Kreislauf zirkulierende transformierte metastasierte Zellen zu erkennen, zu gewinnen, diese außerhalb des Körpers zu kultivieren und für Untersuchungen und andere Anwendungen weiter einzusetzen.

Der Erfindung liegt daher die Aufgabe zugrunde, ein Verfahren zur Verfügung zu stellen, mit dem es auf einfache und ungefährliche Weise in kurzer Zeit möglich ist, aus einem menschlichen oder tierischen Individuum im Blutstrom zirkulierende transformierte Zellen eines Primärtumors zu gewinnen.

Damit verbunden ist die Aufgabe, die so gewonnenen Zellen in Kultur zu vermehren und gegen Antigene auf ihrer Oberfläche gerichtete Antikörper herzustellen, die dann für diagnostische Zwecke eingesetzt werden können.

Die Erfindung beruht auf der Erkenntnis, daß bei der Auftrennung von Vollblut in einer hierzu geeigneten Apherese-Vorrichtung vitale transformierte Zellen in einer Fraktion angereichert werden, die gegebenfalls noch Leukozyten und/oder Lymphozyten enthält.

Die Aufgabe wird somit gelöst durch ein Verfahren zur Isolierung und Kultivierung von im Blutstrom eines Individuums zirkulierenden transformierten Zellen eines Primärtumors, indem man dem Blut mit einer zur Auftrennung in die Blutbestandteile geeigneten Apherese-Vorrichtung eine mit den transformierten Zellen angereicherte Fraktion, die ggf. noch Leukozyten und/oder Lymphozyten enthält, entnimmt und die transformierten Zellen vermehrt, unter der Voraussetzung, daß es sich bei dem Verfahren gemäß der Erfindung nicht um ein therapeutisches Verfahren handelt.

Zur Herstellung der individuumspezifischen, gegen Tumorantigene gerichteten Antikörper bringt man die kultivierten transformierten Zellen mit B-Lymphozyten in Kontakt, um die Produktion von gegen Tumorantigene auf den transformierten Zellen gerichteten Antikörpern zu stimulieren, immortalisiert, vermehrt und selektiert die stimulierten B-Lymphozyten und isoliert und reinigt die Antikörper aus der immortalisierten B-Lymphozytenkultur. Die so gewonnenen Antikörper können polyklonal oder bevorzugt monoklonal sein, wobei man die B-Lymphozyten aus demselben Individuum , aus dem auch die transformierten Zellen isoliert wurden, gewinnt.

Der Organismus wäre prinzipiell in der Lage, die Antikörper, mit seinem Immunsystem selbst herzustellen. Die Zahl der im Blutstrom zirkulierenden transformierten Zellen, und damit die Zahl der entsprechenden Antigene, ist jedoch gerade im für die Diagnostik wichtigen Frühstadium im allgemeinen viel zu gering, um eine ausreichende Immunantwort auslösen zu können. Das erfindungsgemäße Verfahren erlaubt dagegen die Bereitstellung einer großen Menge an tumorspezifischen Antigenen in Form einer Kultur der transformierten Zellen, die sich beliebig vermehren läßt.

Zur Auftrennung des Blutes in seine Bestandteile verwendet man eine hierzu geeignete Vorrichtung, wie sie bei den sogenannten Apherese-Verfahren eingesetzt wird. Im Gegensatz zu den üblichen Auftrennverfahren, wo eine größere Menge Vollblut dem Patienten entnommen und mit einer Zentrifuge in Fraktionen aufgetrennt wird, ist beim Apherese-Verfahren die Auftrennvorrichtung über Leitungen direkt mit dem Blutstrom des Patienten verbunden. Auf diese Weise wird es möglich, das dem Patienten entnommene Blut aufzutrennen, die gewünschte Fraktion abzutrennen und die übrigen Blutbestandteile dem Patienten sofort wieder zurückzugeben. Apherese-Verfahren erlauben somit die quasikontinuierliche Entnahme von bestimmten Blutfraktionen in hohen Mengen, ohne den Patienten zu belasten. So spricht man bei der apheretischen Sammlung von Blutplasma von "Plasmapherese", bei der Sammlung von Leukozyten von "Leukapherese" usw. (US-Patent 5 112 298, US-Patent 5 147 290).

Bisher war man der Annahme, daß die an sich bekannten Apherese-Verfahren nur für die Auftrennung von im Kreislauf ohnehin ständig peripher zirkulierenden weißen Blutkörperchen und anderen, in relativ großen Mengen vorhandenen Blutbestandteilen geeignet seien. Die Erfinder haben nun überraschenderweise festgestellt, daß sich auch die abgesiedelten transformierten Zellen eines Primärtumors in einer bestimmten Zellfraktion anreichern.

Die Auftrennung der Blutbestandteile erfolgt vorteilhaft in einer Zentrifuge mit einem rotierenden Behälter, wie sie üblicherweise auch zur apheretischen Lymphozytenseparation vom Blutplasma, von den Erythrozyten und anderen Blutbestandteilen ohne chemischen Dichtegradienten und anschließender Anreicherung verwendet wird (z.B. Haemonetics V50-1 (sogenannte "Latham-Bowl"), hergestellt von Haemonetics. Inc., USA): Die Zentrifuge selbst hat ein Fassungsvermögen von ca. 100 bis 200 ml Blut.

Solche Zentrifugen wurden bislang insbesondere in einem Verfahren zur Verminderung der Lymphozytenpopulation bei Patienten mit Lymphozytenleukämie in Kombination mit einem extrakorporalen Bestrahlungsverfahren eingesetzt (EP-A 30358). Hierzu wird dem Patienten Blut entnommen und dieses in Gegenwart einer lichtreaktiven chemischen Verbindung, wie z.B. einem Psoralenderivat, das zuvor oral verabreicht nach der Entnahme dem Blut zugegeben wurde, mit UV-Licht bestrahlt. Das Psoralen interkaliert der DNA der Zellen und vermag nach Lichtaktivierung mit der DNA zu reagieren und dabei Onkogene auszuschalten. Anschließend führt man das bestrahlte Blut dem Patienten wieder zu.

Das erfindungsgemäße Verfahren ermöglicht, nur die Fraktion mit den darin angereicherten transfermierten Zellen und den ggf. noch enthaltenen Leukozyten und/oder Lymphozyten schonend aus dem Blut abzutrennen, die übrigen Blutbestandteile werden dem Patienten wieder zurückgegeben. Bevorzugt geschiecht dies kontinuierlich in einem Arbeitsgang. Auf diese Weise wird die Belastung des Patienten so gering wie möglich gehalten, andererseits gelingt es, in kurzer Zeit ausreichende Mengen an transformierten Krebszellen isolieren, die dann entsprechend weiterverarbeitet werden können.

Vor der Blutzuführung wird die Zentrifuge mit einer pharmazeutisch annehmbaren isotonischen Lösung gefüllt, die mit einem gerinnungshemmenden Mittel, z.B. Heparin, versetzt ist, üblicherweise reichen hierfür 1000 bis 2000 Einheiten aus. Für die Separation von transformierten Zellen aus dem Blutstrom können über einen geeigneten, am Zentrifugenausgang angebrachten Bypass-Mechanismus beliebig viele weiße Blutkörperchen mit darunter vermischten transformierten Zellen entnommen wurden, wobei der Fachmann ohne weiteres in der Lage ist. die Leukozytenfraktion zu identifizieren:
Bei Erscheinen einer weißen Bande, die sich deutlich von den roten Erythrozyten abhebt, wird der Bypass der Zentrifuge geöffnet und ca. 40 ml der weißen Blutkörperchen bei einer Zentrifuge mit einem Fassungsvermögen von 120 ml entnommen. Die roten Blutkörperchen, das Plasma und die anderen Blutbestandteile werden dem Körper wieder zurückgegeben.

Die mit den transformierten Zellen angereicherte Fraktion, die ggf. noch Leukozyten und/oder Lymphozyten enthält (im folgenden kurz "Zellfraktion" genannt), wird in eine Kulturlösung überführt. Zur Kultur der transformierten Zellen kann jedes Basiszellkulturmedium eingesetzt werden, welches eine Kohlenstoff- und Stickstoffquelle sowie weitere notwendige anorganische und organische Bestandteile, wie Vitamine, Aminosäuren und Spurenelemente enthält. Bevorzugt sind handelsübliche Medien, wie z.B. RPMI 1640-Medium, ggf. mit einem Supplement wie fötalem Kälberserum ergänzt. Die Kulturbedingungen werden nach den Herstellerangaben eingestellt, üblicherweise werden Standardbedingungen von 5 % CO₂, 37°C und 95 % Luftfeuchtigkeit verwendet.

Bei kleinem Primärtumor, der möglicherweise selbst noch gar nicht diagnostiziert oder lokalisiert wurde oder sich noch im Stadium vor der Metastasenbildung befindet, zirkulieren nur äußerst geringe Mengen an transformierten Zellen im Blut. Die Zugabe von macrophagen-inhibierendem Peptid zur Zellfraktion verhindert, daß in der Zellfraktion die wenigen transformierten Zellen gegenüber dem großen Überschuß an Lymphozyten durch Macrophagen zerstört werden. Zusätzlich hemmt der Zusatz von B- und T-zellenspezitischen Antikörpern (CD-8-, CD-25-, Interleukin- und Interferonspezifische Antikörper) die Vermehrung der Lymphozyten, die schließlich absterben. Über bekannte Zellsorting-Verfahren können alternativ die Leukozyten, Lymphozyten und andere störende Zellen aus der Zellfraktion abgetrennt werden. Die Ansätze werden auf Microtiterplatten verteilt, und schon nach ca. vier Tagen sind die Klone der transformierten Zellen gut sichtbar. Die transformierten Zellen lassen sich aufgrund ihrer Morphologie eindeutig von eventuell noch vorhandenen Lymphozyten unterscheiden und ähneln sehr stark den Primärtumorzellen. Nach ca. einer Woche stehen bereits genug transformierte Zellen für die anschließende Immunisierung zur Verfügung. Für eine Verwendung zu einem späteren Zeitpunkt können die Klone auch eingefroren werden.

Die Krebszellen können dem Körper in modifizierter nicht-pathogener Form als Immunogen wieder zugeführt werden. Hierfür können z.B. isolierte Membranfraktionen, die noch die Tumorantigene tragen, verwendet werden. Hierzu wird mit diesen Antigenen ein anderer Fremdorganismus, z.B. Maus, Kaninchen oder Ziege, oder auch derselbe Organismus, aus dem man die Antigene gewonnen hat, immunisiert. Die nach üblichen Methoden hergestellten polyklonalen oder monoklonalen Antikörper können zur Diagnostik oder Therapie verwendet werden.

Gemäß der Erfindung werden die kultivierten Krebszellen dazu benutzt, aus demselben Organismus isolierte B-Lymphozyten zu stimulieren. Vorteilhaft bedient man sich hierbei der sogenannten vitro-Immunisierung, d.h. die B-Lymphozyten werden außerhalb des Körpers mit den transformierten Zellen in einem geeigneten Medium in Kontakt gebracht. Aus den so stimulierten B-Lymphozyten stellt man Hybridoma-Zellklone her, die zur permanenten Sekretion von Antikörpern in der Lage sind. Neben der Hybridoma-Technik ist auch die Immortalisierung der B-Lymphozyten mit Epstein-Barr-Viren möglich. Die Erfindung bildet somit das körpereigene Immunsystem in technisch kontrollierbarer Weise nach.

In einer Ausführungsform der Erfindung werden die in Kultur vermehrten malignen Zellen zu einem gegebenen Zeitpunkt, jedoch vorzugsweise möglichst früh nach der Entnahme, d.h. nach ca. 8 bis 10 Tagen, mit körpereigenen B-Lymphozyten des Patienten in Kontakt gebracht. Nachdem für die Isolierung der B-Lymphozyten nur etwa 50 ml Blut notwendig sind, können sie auch durch Zentrifugation von Vollblut in einem Dichtegradienten, z.B. einem Ficollgradienten, isoliert werden. Die Entnahme der B-Lymphozyten erfolgt jedoch vorteilhaft mit der erfindungsgemäß verwendeten Auftrennvorrichtung als Lymphozyten-Apherese. Besonders bevorzugt werden die Verfahrenschritte, d.h. die Anlegung der Krebszellenkultur und die Isolierung der B-Lymphozyten, so aufeinander abgestimmt, daß eine in vitro-Immunisierung zu einem möglichst frühen Zeitpunkt nach der Zellentnahme, d.h. nach ca. 8 bis 10 Tagen, erfolgen kann.

Für die in vitro-Immunisierung kann die gesamte Zellfraktion verwendet werden (T- und B-Lymphozyten), da nur die stimulierten B-Lymphozyten die Antikörper sezernieren, wobei die Zellfraktion und die transformierten Zellen in einem verhältnis, bezogen auf die Anzahl der Zellen, bevorzugt von 100:1 bis 1:1 und besonders bevorzugt im Verhältnis von ca. 10:1 eingesetzt werden. Durch Zugabe von Interleukinen wie IL-2, IL-4, IL-5, IL-6 und gamma-lnterferon wird die Stimulierung der antikörperbildenden Zellen begünstigt. Die Konzentration dieser Hilfsstoffe im Immunisierungsansatz beträgt jeweils 0.1 bis 10 mM, bevorzugt ca. 1 mM.

Zur unbegrenzten Vermehrung müssen die stimulierten B-Lymphozyten immortalisiert werden. Dies kann bevorzugt durch Transformation der B-Lymphozyten mit Epstein-Barr-Viren, analog dem Verfahren wie in der deutschen Patentschrift DE 39 28 769 C2 beschrieben, geschehen. Besonders bevorzugt zur Produktion großer Antikörpermengen ist die Herstellung einer Hybridomakultur. Für die Fusion mit den B-Lymphozyten werden je nach Spenderorganismus spezienspezifische Myelomazellen verwendet. Beim Menschen hat sich die Myelomazellinie U-266 für diesen Zweck besonders bewährt. Die B-Lymphozyten werden mit den Myelomazellen in dem optimalen Verhältnis von 1:1 vereinigt. Zur Unterstützung der Zellfusion können Hilfsstoffe wie Polyethylenglycol zugesetzt werden. Anschließend wird der Ansatz zur Selektion auf Hybridomaklone auf Microtiterplatten ausgesät und der Kulturüberstand nach Antikörpern getestet. Die Kulturbedingungen sind wie für Hybridomakulturen üblich.

Durch Epitop-Screening werden Hybridomaklone selektiert, die verschiedene antigene Determinanten auf der Oberfläche der transformierten Zell erkennen, und die Spezifität der Antikörper verifiziert. Die auf diese Weise erhaltenen Hybridomazellinien können zur Gewinnung von Antikörpern weiter vermehrt und/oder für eine spätere Verwendung eingefroren werden.

Die Antikörper werden nach konventionellen Verfahren aus dem Kulturüberstand isoliert und mit üblichen Proteinreinigungs-Methoden, wie Ammoniumsulfatfällung, Gelchromatographie, Ionenaustausch-Chromatographie, Affinitätschromatographie und/oder FPLC und elektrophoretischen Verfahren möglichst bis zur Homogenität gereinigt.

Die extrakorporale in vitro-Immunisierung erfolgt ohne Passagen in Fremdorganismen und liefert große Mengen an Antikörpern, die für den speziellen Tumor aus dem Spenderindividuum spezifisch und somit zur Verwendung für seine Diagnose und seine Bekämpfung in diesem speziellen Individuum optimal geeignet sind. Andererseits stammen die Antikörper aus B-Lymphozyten, die ebenfalls demselben Individuum entnommen wurden. Diese Antikörper sind daher, obgleich außerhalb des Körpers hergestellt, "körpereigene" Proteine, die nach Applizierung im Organismus keine gegen sie gerichtete Immunantwort auslösen, was sonst zu ihrem raschen Abbau und damit zu einer verringerten therapeutischen Wirksamkeit führen könnte.

Die nach dem erfindungsgemäßen Verfahren hergestellten Antikörper umfassen alle Subklassen wie IgG, IgM und IgE, bevorzugt sind Antikörper vom IgG-Typ. Die Antikörper können ferner chemisch oder enzymatisch modifiziert werden, solange ihre physiologische Aktivität, d.h. Erkennung und Bindung an die krebszellen-spezifische antigene Determinante, erhalten bleibt.

An die Antikörper können nach dem konventionellen Glutaraldehyd-Verfahren Immuntoxine, wie z.B. Ricin oder Diphtherie-Toxin gekoppelt werden. Ferner können die Antikörper mit radioaktiven Substanzen, z.B. ¹²⁵J oder Markerenzymen für einen RIA oder ELISA markiert werden.

In einer bevorzugten Ausführungsform werden die Antikörper ohne weitere Modifikationen zur Herstellung eines Arzneimittels verwendet, wobei in einer pharmazeutischen Zusammensetzung eine oder mehrere monoklonale Antikörperarten, die jeweils gegen ein bestimmtes Epitop gerichtet sind, enthalten sein können.

Nach Verabreichung an einen Patienten sind sie in der Lage, an abgesiedelte Tumorzellen zu binden und auf diese Weise Macrophagen oder körpereigene Killerzellen anzulocken, die dann ihrerseits die Krebszellen zerstören.

Die Antikörper werden in einer pharmazeutisch annehmbaren Formulierung zusammen mit hierfür üblichen Trägern, Verdünnern, Stabilisatoren und gegebenenfalls weiteren Hilfstoffen dem Patienten verabreicht. Das Arzneimittel kann in jeder geeigneten Formulierung, bevorzugt in Form einer intravenösen, intramuskulär oder subkutan injizierbaren Lösung vorliegen. Besonders bevorzugt werden die Antikörper nach ihrer Gewinnung lyophilisiert und in einer Einheitsdosis in Ampullen gefüllt und versiegelt. Kurz vor der Verwendung werden Antikörper mit einer für die Injektion geeigneten Trägerlösung gelöst oder suspendiert. Die Trägerlösung, üblicherweise eine sterile, gepufferte wäßrige Lösung, kann in einer eigenen Ampulle zusammen mit den lyophilisierten Antikörpern in einer Arzneimittelpackung beigegeben sein. Der behandelnde Arzt wird hierbei je nach Alter und Zustand des Patienten, nach Art des Tumors und Schwere der Erkrankung eine geeignete Dosierung und Verabreichungsform wählen.

Für die Herstellung eines Arzeimittels zur Tumorbehandlung kann auch bevorzugt die Eigenschaft eines Antikörpers ausgenutzt werden, hochspezifisch sein entsprechendes Antigen zu erkennen und daran zu binden. Ein Wirkstoff, der mit dem Antikörper in geeigneter Weise, d.h. nicht über den für die Antigenerkennung wichtigen Bereich, kovalent verbunden ist, läßt sich somit via Antikörper als Transportvehikel direkt an den gewünschten Wirkort transportieren, um nur dort und nicht unspezifisch im Organismus verteilt seine Wirkung zu entfalten. Durch Ankoppelung von Immuntoxinen, z.B. Ricin, oder Radiotherapeutika an diese Antikörper nach an sich bekannten Verfahren können somit die Tumorzellen zerstört werden.

Durch Verabreichung dieser modifizierten und/oder nichtmodifizierten Antikörper können Tumorzellen erfaßt und zerstört werden, die am Endothel angeheftet waren. Ferner erlaubt dieses Verfahren auch die Zerstörung eventuell nicht operabler solider Tumoranteile oder des gesamten soliden Tumors, wie auch bereits gebildeter Metastasen. Nach operativen Eingriffen können Antikörpergaben über einen entsprechenden Zeitraum vorbeugend gegeben werden, um die Neuansiedlung zirkulierender transformierter Zellen bzw. die Entstehung von Metastasen zu verhindern. Eine Verabreichung kann auch zusammen mit anderen üblichen Arzeimitteln zur Tumorbehandlung wie Cytostatika oder ähnlichen und begleitenden therapeutischen Maßnahmen, Betrahlungen usw. erfolgen.

Für diagnostische Zwecke können diese Antikörper an radioaktive oder nicht-radioaktive Marker gekoppelt werden und in vivo und in vitro der Suche und Markierung von Tumorzellen dienen. Die hohe Selektivität und Bindungsaffinitität der Antikörper erlaubt auf einfache Weise die Anzahl transformierter Zellen im Blutstom eines Patienten in einem in vitro-Testsystem quantitativ und qualitativ zu bestimmen. Dank der Empfindlichkeit eines solchen Tests, mit dem in einer Probe bereits wenige transformierte Zellen nachgewiesen werden können, muß dem Patienten nur wenig Blut entnommen werden, und durch die ständige Verfügbarkeit der Antikörper kann der Test regelmäßig über einen längeren Zeitraum zur Diagnose durchgeführt werden.

Es ist ein wesentliches Kennzeichen der Erfindung, daß die nach diesem Verfahren gewonnenen Produkte, wie z.B. die Kultur der transformierten Zellen, die Hybridomakultur und die Antikörper, hochspezifisch für den betreffenden Organismus sind. Dies bedeutet für die Antikörper, daß sie sich insbesondere hinsichtlich Ihrer nichtantigenerkennenden Bereiche von solchen aus einem anderen Organismus, z.B aus einem anderen Patienten mit derselben Tumorerkrankung, mehr oder weniger stark in ihrer Struktur unterscheiden. Andererseits sind die Antikörper für das jeweilige Individuum praktisch endogen und werden vom Immunsystem nicht als "fremd" erkannt. Das erfindungsgemäße Verfahren ermöglicht, innerhalb eines relativ kurzen Zeitraums und mit vertretbarem Aufwand, für einen bestimmten Patienten, z.B. durch die Verwendung der individuumspezifischen Antikörper, ein speziell für ihn zugeschnittenes Arzneimittel oder Diagnostikum herzustellen, das sich neben seiner spezifischen Wirksamkeit auch durch eine hohe Verträglichkeit auszeichnet.

Dies bedeutet jedoch andererseits nicht, daß die Verwendung von Antikörpern, die aus und für ein bestimmtes Individuum hergestellt wurden, für andere Individuen, insbesondere solchen aus der gleichen Spezies, prinziell ausgeschlossen wäre. Es ist bekannt, daß humane Antikörper bei der Anwendung am Menschen verträglicher sind als Chimären oder tierische Antikörper. Werden die Antikörper zur in vitro-Diagnose eingesetzt. spielt die Verträglichkeit sowieso keine Rolle. Man wird daher bei einem Patienten, bei dem der Verdacht auf eine bestimmte Tumorerkrankung besteht oder eine solche bereits diagnostiziert wurde, zunächst versuchsweise bereits aus einem anderen Patienten gewonnene Antikörper einsetzen. Sprechen diese in gewünschter Weise an und werden sie gut vertragen, ist es nicht mehr notwendig, für diesen Patienten ebenfalls, wie oben beschrieben, eigene Antikörper herzustellen.

Kommt es trotzdem zu Unvertäglichkeitsreaktionen und kennt man die Art des Primärtumor, wird es in vielen Fällen genügen, mit Kulturen transformierter Zellen vom selben Krebstypus, die bereits aus anderen Individuen nach dem erfindungsgemäßen Verfahren isoliert und vermehrt wurden, körpereigene B-Lymphozyten des Patienten zu stimulieren.

Die hieraus hergestellten Antikörper sind, da "körpereigen", hoch verträglich. Erst wenn sie keine ausreichende Spezifität für den individuellen Krebs des Patienten zeigen sollten, ist es nötig. das komplette Isolierungs-, Kultivierungs- und Immunisierungsverfahren gemäß vorliegender Erfindung durchzuführen.

Die vorliegende Erfindung stellt somit ein abgestuftes und individuell anpaßbares Verfahren zur Herstellung eines Mittels zur Verfügung, das zur Diagnose einer bestimmten Krebserkrankung bei einer Vielzahl von Individuen einer bestimmten Säugerspezies eingesetzt werden kann.

In einer bevorzugten Ausführungsform des erfindungsgemäßen Verfahrens werden verschiedene Hybridomazellinien von einem oder mehreren Patienten mit demselben Tumor etabliert und nach solchen monoklonalen Antikörpern selektiert, die spezifische antigene Determinanten auf den Krebszellen unterscheiden. Auf diese Weise läßt sich auch feststellen, ob auf transformierten Zellen, welche aus verschiedenen Patienten mit der gleichen Tumorerkrankung isoliert wurden, identische Determinanten vorkommen. Diese Antikörper können dann zur Diagnose und eventell auch zur Therapie bei anderen Patienten verwendet werden, ohne daß bei ihnen zuerst eine Immunisierung mit körpereigenen B-Lymphozyten durchgeführt werden muß.

Die Antikörper können in modifizierter und nichtmodifizierter Form nach üblichen Methoden, wie oben beschrieben, in pharmazeutische Zusammensetzungen formuliert werden.

Die einfache, schnelle und kostengünstige Durchführbarkeit des Verfahrens erlaubt seinen Einsatz auch bei der Krebsvorsoge oder Früherkennung, ohne daß ein Tumor bereits diagnostiziert wurde. Umsomehr ist es auch geeignet, bei Krebsverdacht im Routinebetrieb auf das Vorhandensein von transformierten Zellen hin zu untersuchen oder bei Vorhandensein eines röntgenologisch erkennbaren soliden Organtumors festzustellen, ob der Tumor bereits maligne Zellen in den Kreislauf gestreut hat oder nicht. Ferner kann bei zweifelhaftem Befund (maligne oder nicht-maligne Zellen) auf das Vorhandensein peripher zirkulierender transformierter Zellen hin untersucht werden. Für diesen Zweck werden die Antikörper bzw. ihre hierfür speziell geeigneten Derivate in einem Diagnose-Kit, vorzugsweise für einen ELISA, bereitgestellt.

Das vorstehende Verfahren ist nicht nur im humanmedizinischen Bereich am Menschen, sondern auch an Säugern allgemein in der Tiermedizin und in verwandten wissenschaftlichen Disziplinen anwendbar. Die beispielhafte Bezugnahme auf einen menschlichen Patienten als Vertreter für einen lebendes Individuum in der Beschreibung soll daher nicht beschränkend ausgelegt werden.

Mit dem erfindungsgemäßen Verfahren ist es möglich, auf ungefährliche Weise ohne Zusatz von Chemikalien oder sonstigen Hilfsmitteln aus einem lebenden Organismus von Mensch und Tier transformierte Zellen für diagnostische Zwecke zu gewinnen.

Das erfindungsgemäße Verfahren bietet insbesondere die folgenden Vorteile:
- Die Gewinnung der transformierten Zellen ist für den Organismus äußerst schonend durchführbar, d.h. es kommt zu keiner vermehrten Freisetzung solcher Zellen in den Blutstrom.
- Die optimale Abstimmung der einzelnen Verfahrensschritte ermöglicht die Erzeugung polyklonaler oder monoklonaler Antikörper in relativ kurzer Zeit und unter niedrigen Kosten.
- Die gewonnenen Antikörper sind individuumspezifisch, d.h. sie stellen für den betreffenden Organismus keine Fremdkörper dar. Es kommt weder zu Unverträglichkeitsreaktion, noch werden die Antikörper im Gegensatz zu anderen Fremdproteinen übermäßig rasch abgebaut, wodurch ihre Effizienz wesentlich erhöht wird.
- Die Antikörper sind tumorspezifisch, d.h. sie erkennen mit hoher Selektivität die spezifischen Antigene des speziellen Tumors aus dem betreffenden Individuum, die Antigenerkennung transformierter Zellen aus anderen Individuen ist jedoch nicht ausgeschlossen.
- Die an die Antikörper gekoppelten pharmazeutischen Wirkstoffe entfalten ihre Aktivität nicht unspezifisch über den Organismus verteilt, sondern im wesentlichen nur am gewünschten Wirkort (celltargeting); unerwünschte Nebenwirkungen werden somit weitgehend vermieden.
- Das aus den Antikörpern hergestellte Arzneimittel kann nicht nur gegen im Blutstrom zirkulierende transformierte Zellen, sondern auch zur Bekämpfung des Primärtumors und eventuell bereits gebildeter Metastasen eingesetzt werden.
- Das aus den Antikörpern hergestellte Arzneimittel wird aus körpereigenen Resourcen gewonnen, das Risiko von Fremdinfektionen, z.B. durch Hepatitis oder AIDS, ist ausgeschlossen.
- Die Gewinnung der für den individuellen Patienten tumorspezifischen Antikörper gelingt mit einem vertretbaren Aufwand, so daß diese nicht nur zur Herstellung patientenspezifischer Arzneimittel, sondern auch für Früherkennung, Diagnostik oder Nachbehandlung nach operativen Eingriffen einsetzbar sind.
- Die Verwendung der aus einem individuellen Organismus gewonnenen Antikörper für andere Organismen als den "Erzeugerorganismus" ist möglich.

Die folgenden Beispiele dienen der Illustration des erfindungsgemäßen Verfahrens.

### Material und Methoden

1. Benötigte Geräte
1.1. Zellkultur:
   sterile Werkbank, CO₂-Inkubator, Wärmeschrank, Wärmeplatte, Fluoreszenzmikroskop, Phasenkontrastmikroskop, Zentrifuge für variable Rotoren. Kühlschränke (4,-20,-80°C), flüssiger Stickstoff, Autoklav
1.2. ELISA:
   Microplattenphotometer, Pipette, 8-Kanal
1.3. Reinigung der monoklonalen Antikörper:
   FPLC, HPLC
1 4. Biochemische Analyse:
   IEF-Gerät, Midget System, Elektroblot. 2dim. Gelelektrophorese. Transformator
1.5. Apherese-Vorrichtung:
   Haemonetics V50-1 (Latham-Bowl), Fassungsvermögen 120 ml, mit Bypass, Haemonetics Inc., USA.

2. Reagenzien
2.1. Gewebekultur:
   Medium RPMI 1640, fötales Kälberserum, L-Glutamin Na-Pyruvat, Phosphatpufferlösung (PBS), pH 7,0, 20mM, HAT-Medium
2.2. Monoklonale Antikörper:
   Anti-CD 8, Anti-CD 25, Anti-lL-2, Anti-IFN gamma (alle erhältlich von Fa. Serva) Rekombinantes IL-2, IL-4, IL-5, IL-6, IFN gamma (alle erhältlich von Fa. Serva) Macrophagen-lnhibierendes-Peptid (Thr-Lys-Pro, Tuftsin-Fragment 1-3), (erhältlich von Fa. Sigma)
2.3. Zellfusion:
   50% Polyethylenglycol 1500 in HEPES-Puffer, 75mM, (50% Gew/Vol).

3. Verfahren
3.1. Isolierung und Vermehrung von Krebszellen mit Hilfe der Apherese:
Unter Verwendung einer mit einem Bypass versehenen Haemonetics V50-Zentrifuge wurden bei zwei Patienten mit unterschiedlichen Tumortypen (Prostatakarzinom, Melanom) nach dem vorstehend beschriebenen Verfahren eine Zellfraktion aus jeweils 120 ml Blut gewonnen.
Bei den Patienten war durch vorherige Operation das histologische Stadium bekannt.
2,4•10⁹ weiße Blutzellen aus der Zellfraktion wurden in ein Röhrchen mit Zellkulturmedium (RPMI 1640) aufgenommen und wie folgt untersucht. Die Zellen wurden über Nacht unter Standardbedingungen (37°C, 5% CO₂, 95% Luftfeuchtigkeit) kultiviert. Als Medium wurde RPMI 1640 mit 10% fötalem Kälberserum unter Zusatz von B- und T-zellspezifischen Antikörpern (CD 8-, CD 25-, IL-2- und IFN gamma-spezifische Antikörper) und Zugabe von Macrophagen-Inhibierendem-Peptid in den folgenden Konzentrationen verwendet:

| | |
|---|---|
| Anti-IFN: Medium | 100 µg/500ml |
| Anti-CD 25: Medium | 100 µg/500ml |
| Anti-CD 8: Medium | 1 mg/500ml |
| Macrophag.-lnhib.-Peptid (MIP): Medium | 50 mg/500ml |

Der Ansatz wurde auf Microtiterplatten mit je 200.000 Zellen in 100 µl pro Well verteilt. Am vierten Tag nach Inokulation wurden die Zellen mit je 100 µl Medium (ohne Antikörper- und MIP-Zusatz) gefüttert. Während der gesamten Kulturzeit wurde ständig mit 5% CO₂ bei 37°C begast. Die Krebszellen waren nach Teilung als Klone bereits nach vier Tagen im Phasenkontrastmikroskop gut sichtbar.
Zur Kontrolle wurde derselbe Ansatz in Medium ohne Zusatz von Antikörpern und Macrophagen-Inhibierendem-Peptid ausplattiert, wobei die Krebszellen erst nach drei Wochen im Phasenkontrastmikroskop als Klone erkennbar waren. Ein Klon des Ansatzes mit zugegebenen Antikörpern und Macrophagen-Inhibierendem-Peptid wurde entnommen und vermehrt, bis eine Zellzahl von 10⁸ erreicht war. Weitere Klone wurden vermehrt und eingefroren.

3.2. Immunisierung:
Die weißen Blutzellen mußten dazu veranlaßt werden, Antikörper gegen krebszellspezifische Antigene zu sezernieren. Hierzu wurden bei einem der Photophoresezyklen 10⁹ weiße Blutzellen entnommen, mit 10⁸ Krebszellen vereinigt und 4 Tage in 100 ml humanem heparinisiertem Plasma in Rollerkultur bei 37°C, 5% CO₂ inkubiert. Durch Zugabe von IL-2, IL-4, IL-5, IL-6 und IFN gamma wurde die Reifung zu antikörperbildenden Zellen stimuliert. Die Konzentrationen betrugen jeweils 100 µg pro 100 ml Immunisierungsansatz.
3.3. Zellfusion:
Die B-Lymphozyten wurden mit Hilfe eines FACS von den übrigen Zellen abgetrennt. Einige Tage vor der Fusion wurden die menschlichen Myelomazellen (U266) in einer Zelldichte von 3•10⁵ Zellen/ml in der logarithmischen Wachstumsphase gehalten. Die Myelomazellen wurden gleichzeitig mit den B-Lymphozyten geerntet und dreimal in PBS gewaschen. Es wurden vier Fusionsansätze vorbereitet, wobei jeweils 5•10⁷ Myelomazellen mit 5•10⁷B-Lymphozyten zusammen zentrifugiert und anschließend mit jeweils 1 ml 50% Polyethylenglycol in HEPES fusioniert wurden.
Hierauf folgte die langsame Zugabe von 10ml RPMI 1640. Der Fusionsansatz wurde in RPMI 1640 gewaschen, 2 Minuten bei 200·g bei RT zentrifugiert, in jeweils 50ml HAT-Medium aufgenommen und zu jeweils 0.1ml pro Well in 96-Well-Microtiterplatten ausgesät. Als Feederzellen wurden humane Macrophagen in einer Anzahl von 10⁴ pro Well eingesetzt.
3.4. Screening:
Der Kulturüberstand der Hybridomazellen wurde mit einem ELISA auf Antikörper getestet. Hierfür wurde ein Zell-ELISA etabliert. Krebszellen wurden mit geeigneten Methoden an die Plastikoberfläche der Microtiterplatten beschichtet, eventuell daran bindende Antikörper über einen zweiten Antikörper detektiert.
ELISA:
Im ersten Screening nach der Fusion wurde nach Klonen gesucht, die Immunglobulin sezernieren.
1. Inkubation von Kulturüberstand in Microtiterplatten zu je 100 µl pro Well über Nacht bei 4° C
2. Dreimal waschen mit PBS
3. Absättigen mit Albumin oder Blotto, 2h, 37°C
4. Dreimal waschen mit PBS
5. Inkubation mit spezifischen Antikörpern gegen humanes Immunglobulin, 2h bei 37°C
6. Inkubation mit einem enzymkonjugierten zweiten Antikörper (Peroxidase oder alkalische Phosphatase) 1h bei 37°C
7. Entwickeln durch Zugabe von Substrat und Messung im Ablesegerät

Die positiven Klone werden nun auf krebszellspezifische Antikörper getestet. Hierzu wurde ein Zell-ELISA etabliert:
1. Herstellung einer Poly-L-Lysinlösung von 1mg/ml in dest. Wasser
2. Inkubation der Microtiterplatten mit Poly-L-Lysin für 15 Minuten
3. Waschen mit Wasser. Lagerung bei RT nach Trocknung
4. Zweimal waschen der Zellen in Suspension in PBS, Zentrifugation bei 400·g
5. Resuspendieren des Zellpellets in PBS zu 10⁵ Zellen pro ml. Einfüllen in die Microtiterplatten, Inkubation 10 min bei RT
Fixierung:
Jeweils 200 µl eines Aceton/Methanolgemisches (50/50 Vol.%) wurden in die Wells der Microtiterplatten eingefüllt und 2 min bei RT inkubiert. Anschließend wurde einmal mit PBS gewaschen.
Die weiteren Schritte wurden wie beim ersten Screening ausgeführt.
3.5. Biochemische Analyse:
Monoklonale Antikörper wurden direkt aus dem Kulturüberstand analysiert. Die Subklassen der leichten und der schweren Kette wurden mit Hilfe eines Subklassentestkits im ELISA bestimmt. Mit Hilfe der isoelektrischen Fokussierung konnte die Monoklonalität der Antikörper sichergestellt werden (Hoffmann et al., Human Genetics, Springer Verlag, 1990, 84,137-146)
3.6. Epitopscreening:
Es wurden Krebszellen kultiviert, 10⁷ Zellen wurden entnommen, dreimal mit PBS gewaschen und in zwei Fraktionen aufgeteilt. Eine Fraktion wurde homogenisiert und in der nativen Gelelektrophorese (pH 8,8, 9% Acrylamid im Trenngel und 3% Acrylamid im Sammelgel) analysiert, die andere wurde denaturiert (Harnstoff, SDS) und mit der eindimensionalen und zweidimensionalen Gelelektrophorese (Waldinger et al., Electrophoresis, 1986) aufgetrennt. Nach anschließendem Westernblot wurde mit dem Kulturüberstand detektiert. Die erkannte Bande bzw. der erkannte Spot wurde extrahiert und ansequenziert und so das tumorspezifische Antigen identifiziert. Als Kontrolle wurden Proben anderer Gewebe und weiße Blutzellen verwendet (E. Harlowe, D. Lane Antibodies, A Laboratory Manual Cold Spring Harbor Laboratory, 1988; D.Baron, U.Hartlaub, Humane monoklonale Antikörper, Gustav Fischer Verlag, Stuttgart, New York 1987).
4. Ergebnisse
In beiden Fällen (Prostatakarzinom, Melanom) waren bereits nach vier Tagen in ca. 30 % der Wells der Zellkulturplatten Klone transformierter Zellen im Phasenkontrast sichtbar.
Als Kontrolle diente die Zellfraktion eines Patienten mit einer nicht-malignen Erkrankung (autoimmune Regulationsstörung der Lymphozyten), bei dem die weißen Blutkörperchen aus anderen Gründen außerhalb des Körpers behandelt werden mußten. In diesem Falle wurden keinerlei Zellteilungen festgestellt.
In analoger Weise wurden die folgenden transformierten Zellinien isoliert und hieraus Hybridomakulturen hergestellt, die entsprechende Antikörper sezernierten:
- Mammakarzinom
- B-Zell-Lymphom
- Bronchialkarzinom
- Nieren-Adenokarzinom

## Patentansprüche

1. Verfahren zur Isolierung und Kultivierung von im Blutstrom eines Individuums zirkulierenden transformierten Zellen,
**dadurch gekennzeichnet**, daß dem Blut mit einer zur Auftrennung in die Blutbestandteile geeigneten Apherese-Vorrichtung eine mit durch einen Primärtumor ausgelösten transformierten Zellen angereicherte Zellfraktion, die ggf. noch Leukozyten und/oder Lymphozyten enthält, entnommen wird und daß die in der betreffenden Zellfraktion enthaltenen transformierten Zellen vermehrt werden, unter der Voraussetzung, daß es sich bei dem beanspruchten Verfahren nicht um ein therapeutisches Verfahren handelt.

2. Verfahren nach Anspruch 1,
dadurch gekennzeichnet,
daß man zur Vermehrung der transformierten Zellen das Wachstum und/oder die Funktion der Lymphozyten und/oder Leukozyten, die ggf. in der mit den transformierten Zellen angereicherten Fraktion enthalten sind, inhibiert.

3. Verfahren nach Anspruch 2,
dadurch gekennzeichnet,
daß man zur Inhibierung Macrophagen-Inhibierendes-Peptid, B-lymphozytenspezifische und/oder T-zellenspezifische Antikörper zugibt.

4. Verfahren nach Anspruch 2,
dadurch gekennzeichnet,
daß man die Leukozyten und/oder Lymphozyten aus der mit den transformierten Zellen angereicherten Fraktion durch ein Zellsorting-Verfahren abtrennt.

5. Verfahren nach einem oder mehreren der Ansprüche 1 bis 4,
dadurch gekennzeichnet,
daß man transformierte Zellen, ausgewählt aus Mammakarzinom, B-Zell-Lymphom, Nieren-Adenokarzinom, Bronchialkarzinom, Prostatakarzinom und Melanom isoliert und kultiviert.

6. Verwendung der nach einem Verfahren nach einem der Ansprüche 1 bis 5 isolierten und kultivierten transformierten Zellen zur Herstellung individuumsspezifischer, gegen Tumorantigene gerichteter Antikörper, wobei man B-Lymphozyten mit den transformierten Zellen zur Produktion von Antikörpern, die gegen Tumorantigene auf den transformierten Zellen gerichtet sind, stimuliert, die stimulierten B-Lymphozyten immortalisiert, vermehrt und selektiert, und die Antikörper aus der immortalisierten B-Lymphozytenkultur isoliert und reinigt, wobei man die B-Lymphozyten aus demselben Individuum, aus dem auch die transformierten Zellen isoliert wurden, gewinnt.

7. Verwendung nach Anspruch 6, wobei man zur Immortalisierung die stimulierten B-Lymphozyten mit einer speziesspezifischen Myelomakultur unter Erhalt einer die Antikörper produzierenden Hybridomazellinie fusioniert und selektiert.

8. Verwendung nach Anspruch 7, wobei man eine individuumsspezifische Hybridomazellinie, ausgewählt aus Mammakarzinomhybridom, B-Zell-Lymphomhybridom, Nieren-Adenokarzinomhybridom, Bronchialkarzinomhybridom, Prostatakarzinomhybridom und Melanomhybridom, herstellt.

9. Verwendung nach Anspruch 6, wobei man die stimulierten B-Lymphozyten durch Transformation mit Epstein-Barr-Viren immortalisiert.

## Claims

1. Process for the isolation and culturing of transformed cells circulating in the bloodstream of an individual, characterized in that a cell fraction enriched with transformed cells, which optionally still contains leucocytes and/or lymphocytes, induced by a primary tumour is removed from the blood with an apheresis device suitable for separating it into the blood constituents and in that the transformed cells contained in the cell fraction concerned are proliferated, on condition that the claimed process is not a therapeutic process.

2. Process according to Claim 1, characterized in that, for the proliferation of the transformed cells, the growth and/or the function of the lymphocytes and/or leucocytes which are optionally contained in the fraction enriched with the transformed cells are/is inhibited.

3. Process according to Claim 2, characterized in that, for the inhibition, macrophage-inhibiting peptide, B lymphocyte-specific and/or T cell-specific antibodies is/are added.

4. Process according to Claim 2, characterized in that the leucocytes and/or lymphocytes are separated from the fraction enriched with the transformed cells by a cell-sorting process.

5. Process according to one or more of Claims 1 to 4, characterized in that transformed cells selected from the group consisting of breast carcinoma, B-cell lymphoma, kidney adenocarcinoma, bronchial carcinoma, prostate carcinoma and melanoma are isolated and cultured.

6. Use of the transformed cells isolated and cultured by a process according to one of Claims 1 to 5 for the production of individual-specific antibodies directed against tumour antigens, where B lymphocytes are stimulated with the transformed cells to produce antibodies which are directed against tumour antigens on the transformed cells, the stimulated B lymphocytes are immortalized, proliferated and selected, and the antibodies are isolated from the immortalized B-lymphocyte culture and purified, the B lymphocytes being obtained from the same individual from which the transformed cells were isolated.

7. Use according to Claim 6, where, for immortalization, the stimulated B lymphocytes are fused with a species-specific myeloma culture with retention of a hybridoma cell line producing the antibodies and selected.

8. Use according to Claim 7, where an individual-specific hybridoma cell line selected from the group consisting of mammary carcinoma hybridoma, B-cell lymphoma hybridoma, kidney adenocarcinoma hybridoma, bronchial carcinoma hybridoma, prostate carcinoma hybridoma and melanoma hybridoma is prepared.

9. Use according to Claim 6, where the stimulated B lymphocytes are immortalized by transformation with Epstein-Barr viruses.

## Revendications

1. Procédé pour isoler et cultiver des cellules transformées circulant dans le courant du flux sanguin d'un individu, caractérisé par le fait que l'on prélève du sang, à l'aide d'un dispositif à éphérèse qui est approprié à séparer des éléments constitutifs ou composantes du sang, une fraction de cellules qui est enrichie avec des cellules transformées déclenchées par une tumeur primaire, et qui contient éventuellement encore des leucocytes et/ou des lymphocytes, et que les cellules transformées qui sont contenues dans ladite fraction de cellule sont enrichies, sous la réserve que le procédé revendiqué ne soit pas considéré comme étant un procédé thérapeutique.

2. Procédé selon la revendication 1, caractérisé par le fait que pour l'enrichissement des cellules transformées, on inhibe la croissance et/ou la fonction des lymphocytes et/ou des leucocytes qui sont éventuellement contenus dans les fractions enrichies en les cellules transformés.

3. Procédé selon la revendication 2, caractérisé par le fait que pour l'inhibîtion, on ajoute un peptide macrophagé à action d'inhibîtion, des anticorps spécifiques aux B-lymphocytes et/ou des anticorps spécifiques aux cellules T.

4. Procédé selon la revendication 3, caractérisé par le fait que par un procédé de triage des cellules, on sépare les leucocytes et/ou les lymphocytes de la fraction qui a été enrichie en les cellules transformées.

5. Procédé selon l'une ou plusieurs des revendications 1 à 4, caractérisé par le fait que l'on isole et cultive les cellules transformées choisies parmi le carcinome mammaire, le lymphome de cellule B, l'adénocarcinome du rein, le carcinome bronchial, le carcinome prostatique et le mélanome.

6. Mise en oeuvre de cellules transformées, isolées et cultivées selon un procédé tel qu'indiqué dans l'une des revendications 1 à 5, en vue de la préparation d'anticorps spécifiques à l'individu et servant d'antigène tumoral, du type dans lequel on stimule des lymphocytes B avec les cellules transformées pour produire des anticorps susceptibles d'agir comme antigène tumoral sur les cellules transformées, on immortalise, on multiplie et on sélectionne les lymphocytes B stimulés, lesdits anticorps étant isolés de la culture immortalisée de lymphocytes B et étant nettoyés, alors que l'on obtient les lymphocytes B du même individu duquel ont été également isolées les cellules transformées.

7. Mise en oeuvre selon la revendication 6, dans laquelle, pour l'immortalisation, les lymphocytes B stimulés sont fusionnés avec une culture de myélone spécifique au genre et obtention d'une ligne de cellules hybrides qui produisent les anticorps, et sont ensuite sélectionnées.

8. Mise en oeuvre selon la revendication 7, dans laquelle on prépare une ligne de cellules hybrides, spécifique à l'individu, sélectionnée parmi le carcinome hybride mammaire, le lymphe hybride des cellules B, l'adénocarcinome hybride rénal, le carcinome hybride bronchial, le carcinome hybride prostatique et l'hybride de mélanome.

9. Mise en oeuvre selon la revendication 6, dans laquelle on immortalise les lymphocytes B stimulés par transformation avec des virus Epstein-Barr.
